# EUROPEAN PATENT SPECIFICATION

(11) **EP 1 116 713 B1**
(45) Date of publication and mention of the grant of the patent: **17.02.2010**
(21) Application number: 99940638.2
(22) Date of filing: 03.09.1999
(51) Int. Cl.: C07C 275/40, C07C 317/42, C07C 311/47, B41M 5/333, B41M 5/155

(54) **COLOR-DEVELOPING COMPOUND AND RECORDING MATERIAL**
FARBENTWICKLUNGSVERBINDUNG UND AUFZEICHNUNGSMATERIAL
COMPOSÉ DÉVELOPPEUR DE COULEUR ET MATÉRIAU D'ENREGISTREMENT

(30) Priority: 04.09.1998 JP 25067198
(43) Date of publication of application: 18.07.2001
(73) Proprietor: Chemipro Kasei Kaisha, Ltd., Hyogo (JP)
(72) Inventor: KABASHIMA, Kazuo, Yokohama-shi Kanagawa 230-0075 (JP); IWAYA, Tetsurou, Kawasaki-shi Kanagawa 211-0041 (JP)
(74) Representative: Strehl Schübel-Hopf & Partner
(86) International application number: PCT/JP1999/004776
(87) International publication number: WO 2000/014058

(56) References cited:
- EP-A- 0 535 887
- EP-A- 0 633 145
- WO-A2-98/03518
- JP-A- 2 141 288
- JP-A- 4 069 278
- JP-A- 5 068 873
- JP-A- 6 507 661
- JP-A- 9 296 196
- JP-A- 57 112 363
- JP-A- 59 030 919
- JP-A- 60 104 055
- JP-A- 60 226 854

## Description

The present invention relates to a novel urea-urethane compound. The present invention relates also to a novel color-producing composition obtained by using the urea-urethane compound.

The color-producing composition of the present invention is useful as a color-producing composition for recording materials, which use a recording energy such as heat, pressure or the like, and the present invention relates to, in particular, a color-producing composition capable of giving an improved storage stability to an uncolored portion (an original recording material surface) and a developed color image.

### BACKGROUND ART

Various chemical color-producing systems which use a recording energy such as heat, pressure or the like have been known. Of these systems, color-producing systems usually composed of a two-component color-producing system consisting of a colorless or light-colored dye precursor and a developer capable of causing color development on contact with the dye precursor have been known since early times and are commonly utilized in recording materials. There are, for example, pressure-sensitive recording materials which record using pressure, heat-sensitive recording materials which record using heat, and light-sensitive recording materials which record using light.

Pressure-sensitive recording materials have been generally used in the planar form, similar to paper. In general, the pressure-sensitive recording material is obtained by dissolving a dye precursor in a suitable solvent, emulsifying the resulting solution to several microns, and forming the emulsion into microcapsules. A first layer (also referred to herein as upper paper) of paper obtained by coating a substrate with the microcapsules and a second layer (also referred to herein as under paper) of paper obtained by coating another substrate with a developer layer containing a developer are placed one upon the other so that the microcapsule-coated surface and the developer-coated surface face each other. When a pressure is applied to the resulting assembly by writing, striking or the like, the microcapsules are destroyed to release the contents including the dye precursor. The dye precursor transfers to the developer layer to come into contact with the developer, so that color development reaction occurs, resulting in recording of an image.

In recent years, a heat-sensitive recording method comprising recording by means of heat energy has been often adopted in various information machines such as facsimiles, printers, recorders and the like. A heat-sensitine recording material used in the heat-sensitive recording method has many excellent characteristics such as a high whiteness, appearance and feel which are similar to those of ordinary planar paper, and having an excellent aptitude for recording, for example, a high color development sensitivity. The heat-sensitive recording method is advantageous, for example, in that an apparatus used in the method is small, requires no maintenance and produces no noise. Therefore, the range of use of the heat-sensitive recording method have been increased in various fields of, for instance, recorders for measurement, facsimiles, printers, terminals of computer, labels, and automatic vending machines for railroad tickets or the like.

In the heat-sensitive recording method, a recording material obtained by forming on a substrate a color-producing layer containing a two-component color-producing composition is mainly used, and the components of the heat-sensitive composition are brought into contact with each other by treating the recording material with heat supplied as the recording energy from a thermal head, a hot stamp, laser beams or the like. Many compositions used as the color-producing composition are those obtained by using a colorless or light-colored, electron-donating dye precursor (in particular, a leuco dye) and an acidic developer such as a phenolic compound. An example of a recording material obtained by using a leuco dye is thermal paper obtained by using a combination of Crystal Violet lactone and 4,4'-isopropylidenediphenol (bisphenol A) as a heat-sensitive color-producing composition (see U.S. Patent 3539375, etc.).

As the dye precursor and developer used in each of the recording methods described above, an electron-donating compound and an electron-accepting compound, respectively, are mainly used. This is because the electron-donating compound and the electron-accepting compound have, for example, the following excellent characteristics: the dye precursor as electron-donating compound and the developer as electron-accepting compound come into contact with each other to give a nearly instantaneous developed color image with a high density; and a nearly white appearance can be obtained and various hues such as red, orange, yellow, green, blue, black, etc. can be obtained. However, the developed color image obtained is so poor in chemical resistance that the record disappears easily on contact with a plasticizer contained in a plastic sheet or an eraser, or a chemical contained in food or cosmetics. Also the developed color image is so poor in record storage stability that the record fades or, worse yet, disappears when exposed to sunlight for a relatively short period of time. Therefore, color-producing compositions comprising the dye precursor and the developer are limited in their use to a considerable extent, and their improvement is eagerly desired.

In recent years, phenolic compounds represented by bisphenol A are considered unsuitable for use because they are likely to be endocrine disrupters, and hence a non-phenolic developer is preferred.

For fulfilling such a request, for example, JP-A-59-115887 and U.S. Patent 4521793 disclose recording materials comprising a combination of color-producing compositions comprising an aromatic isocyanate and an imino compound, as recording materials having a high shelf stability. These references disclose various recording materials in which the two color-producing compositions are brought into contact with each other to be reacted, by application of recording energy such as heat, pressure, light or the like. The references describe the fact that various colors such as red, orange, yellow, light brown, dark brown, etc. can be developed by properly selecting the color-producing compositions. However, in the inventions disclosed in the references, the development of a black color is not yet sufficient and is eagerly desired in the case of recording materials commonly used at present.

JP-A-8-2111 and JP-A-8-2112 disclose heat-sensitive recording materials having a color-producing layer containing a colorless or light-colored dye precursor and a urea compound, as heat-sensitive recording materials obtained by using a non-phenolic developer. These recording materials, however, give a low coloring density and have an insufficient shelf stability.

JP-A-5-116459 discloses a heat-sensitive recording material having a heat-sensitive color-producing layer containing a colorless or light-colored dye precursor and a sulfonylurea compound. This recording material, however, gives a low whiteness and has an insufficient shelf stability.

US 4,388,238 discloses a urea-urea compound of a particular structure wherein the oxygen group of the urethane group is attached to the carbon atom of an alkyl or cycloalkyl group linked with the urethane group.

WO 98/03518 discloses a urea-urethane compound having the structural formula (CH₃)₃C-OCONH-(CH₂)ₙ-NHCONH-(CH₂)ₘ-NHCOO-C(CH₃)₃ as an intermediate compound.

US 4,566,981 discloses a urea-urethane compound having a fluoroaliphatic radical.

JP-A-59-030919 discloses a perfluoroalkyl group-containing urea-urethane compound.

JP-A-05-068873 discloses specific urea-urethane compounds.

EP 0 138 769 A2 discloses diurethane diurea compounds wherein nitrogen atoms at the molecule ends are linked with carbon atoms of alkyl groups bound to the N atoms.

US 5,854,183 discloses a grease composition containing a urea-urethane thickener.

WO 92/020747 discloses an anti-fouling composition in which a urea-urethane compound is used as an anti-fouling agent-modified adduct.

### DISCLOSURE OF THE INVENTION

The present invention is intended to provide a novel urea-urethane compound that exhibits excellent performance characteristics when used as developer in a color-producing composition.

The present invention is also intended to provide a novel color-producing composition excellent in image preservability and coloring density.

In addition, the present invention relates to a novel color-producing composition possessing further improved performance characteristics by virtue of the addition of various additives to a urea-urethane compound and a dye precursor.

The present inventors earnestly investigated the synthesis of various compounds for color-producing composition and consequently found that specific compounds exhibit surprisingly excellent performance characteristics, whereby the present invention has been accomplished. Furthermore, the present inventors found that specific compounds exhibit surprisingly excellent performance characteristics in combination with a dye precursor, whereby the present invention has been accomplished.

That is, the present invention is as follows.

A first aspect of the invention is directed to a urea-urethane compound represented by the following formula (III) : 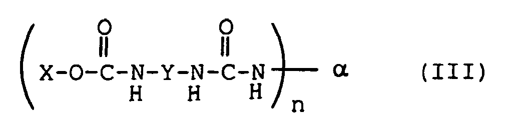 wherein X is a phenyl group, Y is a tolylene group or a methylenediphenyl group represented by -Ph-CH₂₋Ph-, α is a residue having a valence of 2 represented by -Ph- or -Ph-ε-Ph-, and n is 2, wherein -Ph- is a phenylene group and -ε- is a group selected from -SO₂-, -O-, -CH₂- and -CONH-.

A second aspect of the invention is directed to a color-producing composition comprising a developer comprising a urea-urethane compound, and a colorless or light-colored dye precursor, wherein said develper is a urea-urethane compound according to the first aspect of the invention.

A further aspect of the invention is directed to a color-producing composition according to the second aspect of the invention, wherein the developer further comprises an acidic developer.

### BRIEF DESCRIPTION OF DRAWINGS

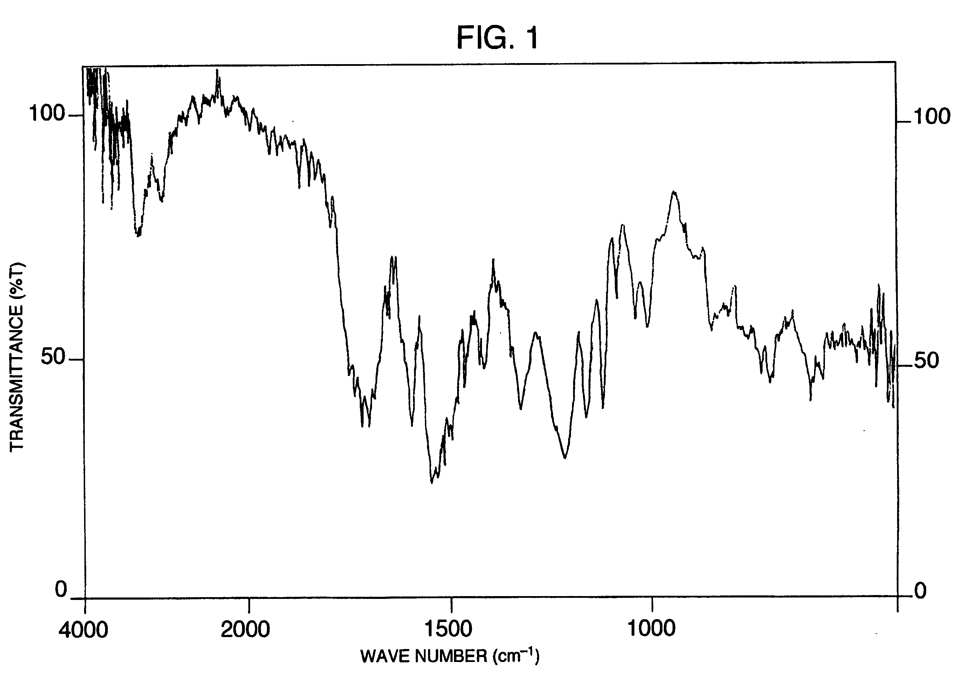

Fig. 1 is an IR spectrum of the white crystals obtained in Example 1.

### BEST MODE FOR CARRYING OUT THE INVENTION

The present invention is explained below in detail.

The urea-urethane compound represented by formula (III) of the present invention is a novel compound. This novel compound is useful in the case of, for example, recording materials used by means of recording energy such as heat or pressure.

A process for producing the urea-urethane compound represented by the formula (III) of the present invention is not limited. This compound can be obtained, for example, by reacting an OH group-containing compound of the general formula (IX) with an isocyanate compound of the general formula (XII) and an amine compound of the following general formula (XIII) according to, for instance, the reaction formula (C) or (D) shown below:

α-(NH₂)ₙ (XIII)

wherein α is a residue having a valence of 2, and n is an integer of 2,

X-OH (IX)

OCN-Y-NCO (XII),

wherein X in formula (IX) is a phenyl group, and Y i formula (XII) is a tolylene group or a methylenediphenyl group represented by -Ph-CH₂-Ph-.

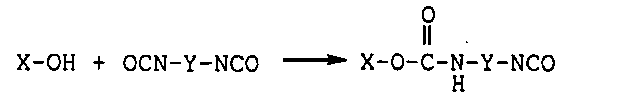

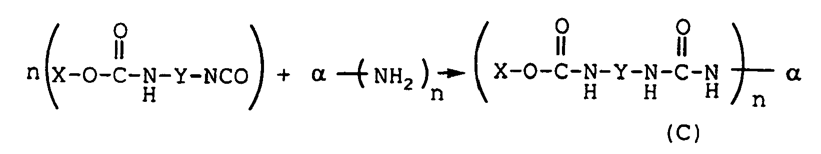

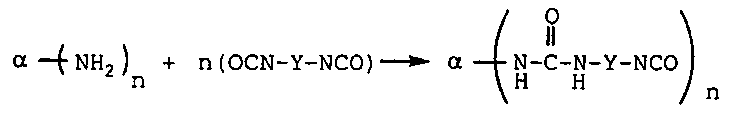

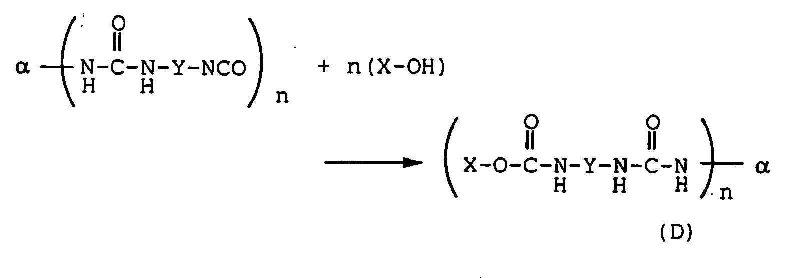

The OH group-containing compound of the general formula (IX) is phenol.

The isocyanate compound of the general formula (XII) is a toluene diisocyanate or diphenylmethane diisocyanate. This compound includes, for example, 2,4-toluene diisocyanate, 2,6-toluene diisocyanate or diphenylmethane diisocyanate.

The amine compound of the general formula (XIII) has two amino groups. This compound includes, for example, 4,4'-diaminobenzanilide, diaminodiphenyl ether, 4,4'-diaminodiphenyl ether, 3,3'-diaminodiphenyl ether, 3,4'-diaminodiphenyl ether, 9,4'-diaminodiphenylmethane, 3,4'-diaminodiphenylmethane, 4,4'-diaminodiphenyl sulfone, 3,3'-diaminodiphenyl sulfone, 3,4'-diaminodiphenyl sulfone, 3,3'-diaminodiphenylmethane, o-phenylenediamine, m-phenylenediamine and p-phenylenediamine.

The urea-urethane compound of the present invention may be obtained by mixing the isocyanate with the corresponding reactants in an organic solvent or without a solvent, reacting them, and then collecting the resulting crystals by filtration. As each of the reactants, one or more compounds may be used depending on purposes. As the solvent, any solvent may be used so long as it does not react with an isocyanate group and the functional groups of the reactants. The solvent includes, for example, aliphatic hydrocarbons, alicyclic hydrocarbons, aromatic hydrocarbons, chlorinated aliphatic hydrocarbons, chlorinated aromatic hydrocarbons, chlorinated alicyclic hydrocarbons, and ketones. Especially preferable are methyl ethyl ketone or toluene, which dissolve the isocyanate and in which the reaction product has a low solubility. The reaction product obtained by the above reaction procedure is not always a single compound, but is obtained as a mixture of compounds different in the position of a substituent, in some cases.

Specific examples of the urea-urethane compound of the first aspect of the present invention are the following compounds ((E-19) to (E-28) and (E-35) to (E-37)).

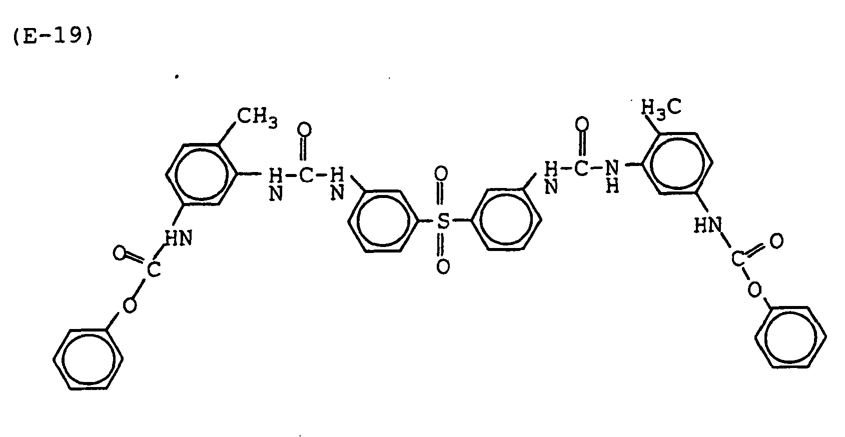

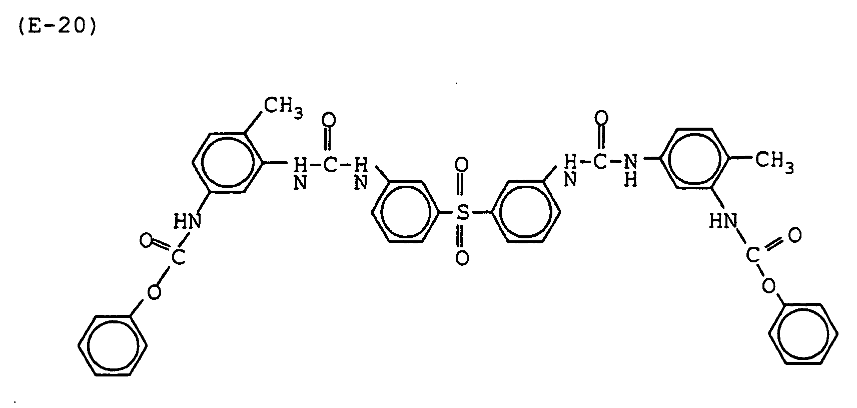

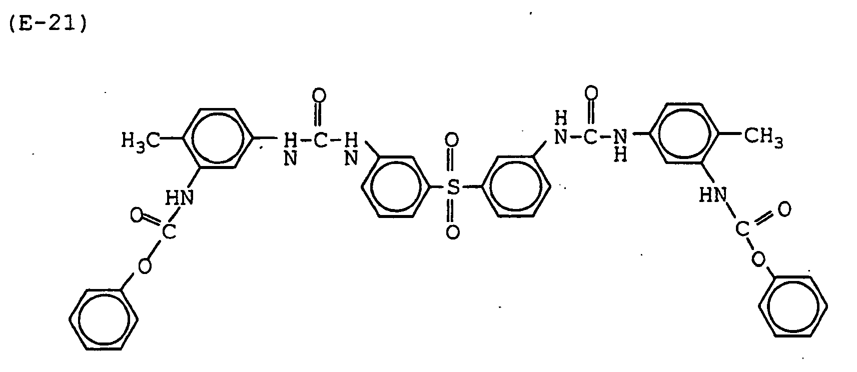

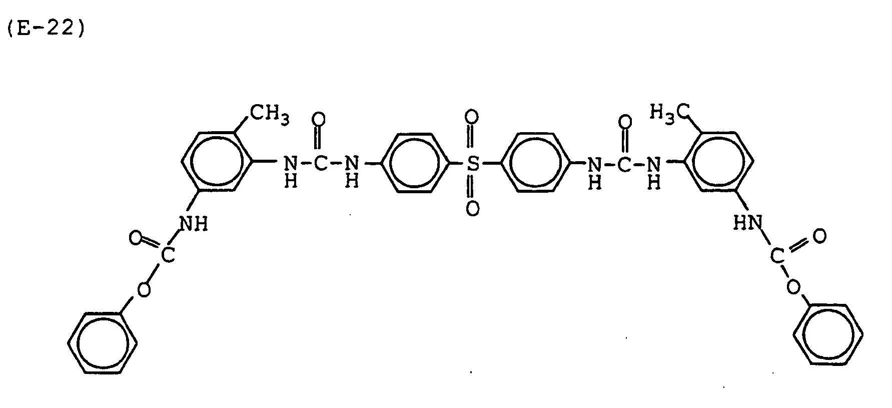

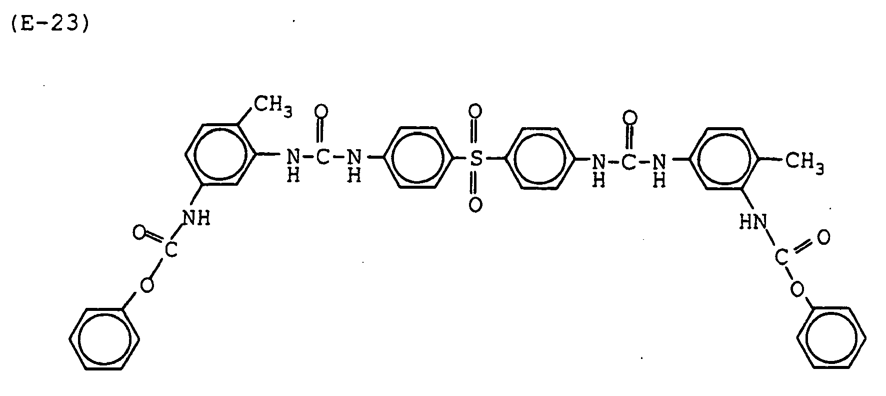

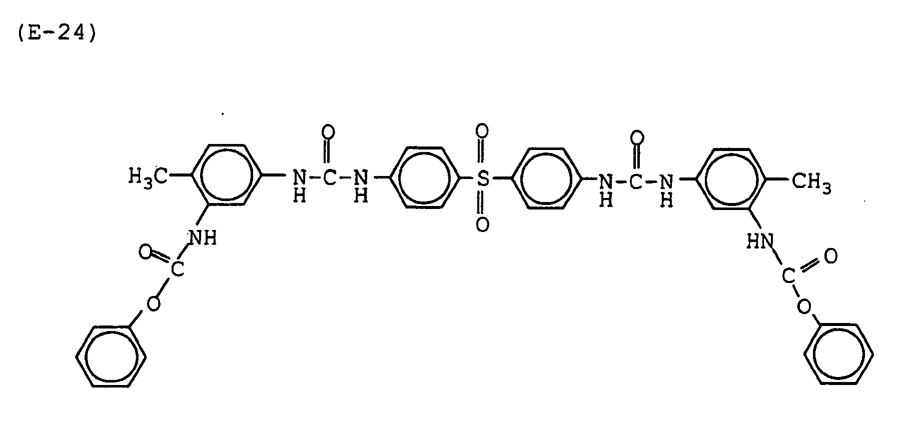

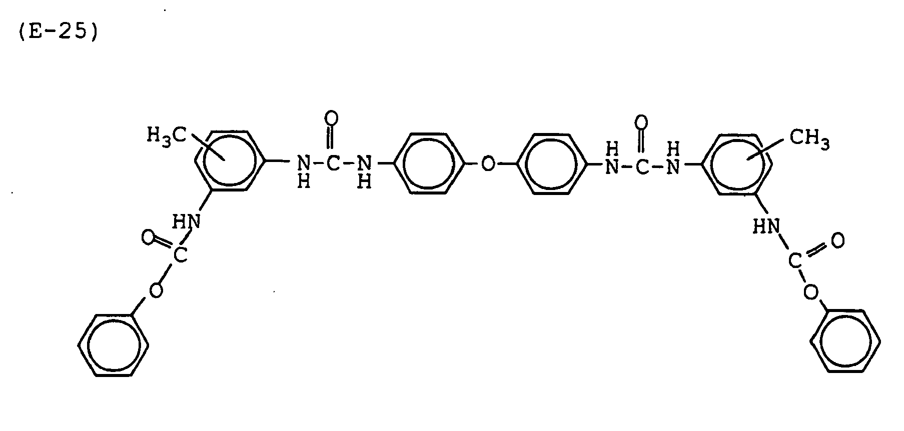

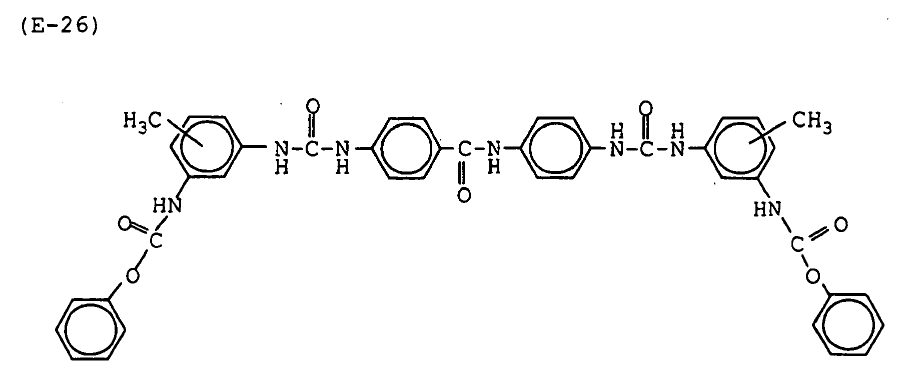

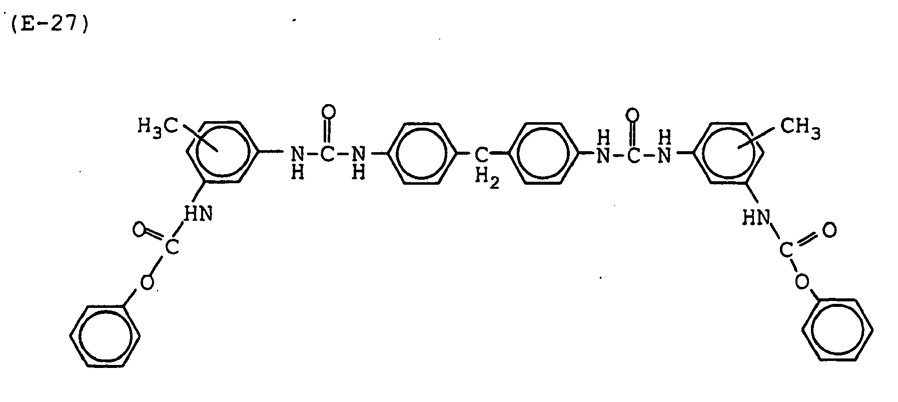

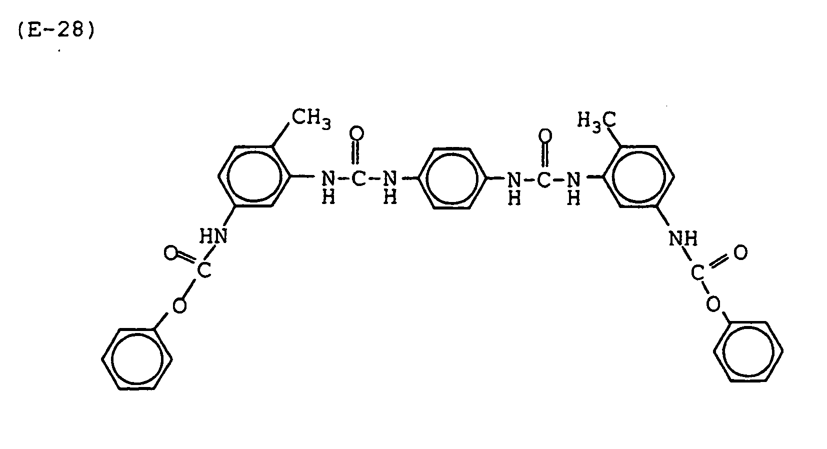

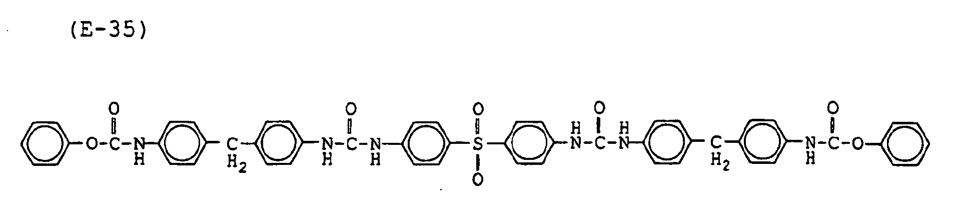

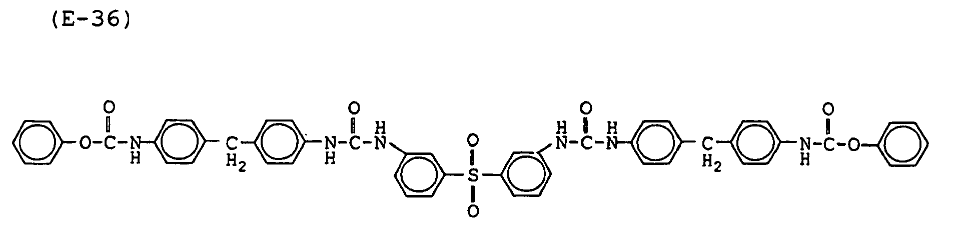

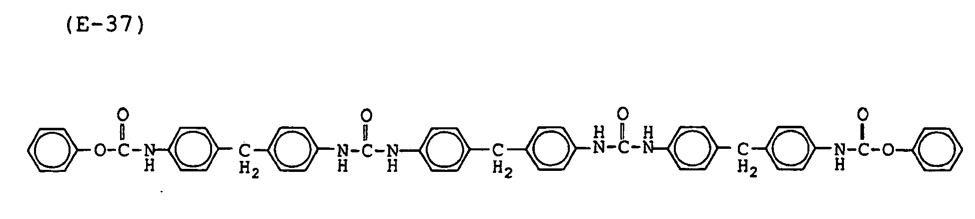

The urea-urethane compound of the present invention is useful in the case of, for example, recording materials.

The urea-urethane compound used as a developer in the second aspect of the present invention refers to a compound as defined in claim 1.

It has been known that compounds having one or more urea groups have color-developing effect, but they have not been practical because they give a low coloring density and have an insufficient shelf stability. However, surprisingly, a urea-urethane compound of the present invention is an excellent developer for a colorless or light-colored dye precursor, and a color-producing composition comprising the urea-urethane compound and the dye precursor and a recording material obtained by using the color-producing composition give a high coloring density and have an excellent shelf stability.

Although a mechanism by which such a urea-urethane compound exhibits an excellent color-developing effect is unknown, it is conjectured that the effect is due to the interaction between the urea group(s) and the urethane group(s) in the molecule.

When used in a heat-sensitive recording material, the urea-urethane compound is preferably one that has a melting point. The melting point ranges preferably from 40°C to 500°C, in particular, from 60°C to 300°C.

A process for synthesizing the urea-urethane compound used as developer in the second aspect of the present invention has been described above.

Epecially preferable examples of the starting isocyanate are toluene diisocyanates. Of the toluene diisocyanates, 2.4-toluene diisocyanate is preferable. Besides 2.4-toluene diisocyanate, mixtures of 2.4-toluene diisocyanate and 2,6-toluene diisocyanate are generally on the market and available at a low price and may also be used as the starting isocyanate.

The urea-urethane compound used as developer in the present invention is usually a colorless or light-colored compound that is solid at ordinary temperature. When used in a heat-sensitive recording material, the melting point of urea-urethane compound used as developer in the present invention is preferably a compound having a melting point, and its melting point ranges preferably from 40°C to 500°C, in particular, from 60°C to 300°C.

For producing a recording material by using the urea-urethane compound as developer, the urea-urethane compound of one kind or, if necessary, a combination of the urea-urethane compounds of two or more kinds may be used.

The colorless or light-colored dye precursor used in the present invention is a compound well known as a color former used in pressure-sensitive recording materials and heat-sensitive recording materials and is not particularly limited. As the dye precursor, electron-donating dye precursors are preferable. Leuco dyes, in particular, triarylmethane type leuco dyes, fluoran type leuco dyes, fluorene type leuco dyes, diphenylmethane type leuco dyes and the like are more preferable. Typical examples of the dye precursor are given below.

### (1) Triarylmethane type compounds

3,3-bis(p-dimethylaminophenyl)-6-dimethylaminophthalide (Crystal Violet lactone), 3,3-bis(p-dimethylaminophenyl)phthalide, 3-(p-dimethylaminophenyl)-3-(1,2-dimethylindol-3-yl)phthalide, 3-(p-dimethylaminophenyl)-3-(2-methylindol-3-yl)phthalide, 3-(p-dimethylaminophenyl)-3-(2-phenylindol-3-yl)phthalide, 3,3-bis(1,2-dimethylindol-3-yl)-5-dimethylaminophthalide, 3,3-bis(1,2-dimethylindol-3-yl)-6-dimethylaminophthalide, 3,3-bis(9-ethylcarbazol-3-yl)-5-dimethylaminophthalide, 3,3-bis(2-phenylindol-3-yl)-5-dimethylaminophthalide, 3-p-dimethylaminophenyl-3-(1-methylpyrrol-2-yl)-6-dimethylaminophthalide, etc.

### (2) Diphenylmethane type compounds

4,4'-bis-dimethylaminophenylbenzhydryl benzyl ether, N-halophenylleucoauramines, N-2,4,5-trichlorophenylleucoauramine, etc.

### (3) Xanthene type compounds

Rhodamine B anilinolactam, Rhodamine B-p-chloroanilinolactam, 3-diethylamino-7-dibenzylaminofluoran, 3-diethylamino-7-octylaminofluoran, 3-diethylamino-7-phenylfluoran, 3-diethylamino-7-chlorofluoran, 3-diethylamino-6-chloro-7-methylfluoran, 3-diethylamino-7-(3,9-dichloroanilino)fluoran, 3-diethylamino-7-(2-chloroanilino)fluoran, 3-diethylamino-6-methyl-7-anilinofluoran, 3-(N-ethyl-N-tolyl)amino-6-methyl-7- anilinofluoran, 3-piperidino-6-methyl-7-anilinofluoran, 3-(N-ethyl-N-tolyl)amino-6-methyl-7-phenethylfluoran, 3-diethylamino-7-(4-nitroanilino)fluoran, 3-dibutylamino-6-methyl-7-anilinofluoran, 3-(N-methyl-N-propyl)amino-6-methyl-7-anilinofluoran, 3-(N-ethyl-N-isoamyl)amino-6-methyl-7-anilinofluoran, 3-(N-methyl-N-cyclohexyl)amino-6-methyl-7-anilinofluoran, 3-(N-ethyl-N-tetrahydrofuryl)amino-6-methyl-7-anilinofluoran, etc.

### (4) Thiazine type compounds

benzoylleucomethylene blue, p-nitrobenzoylleucomethylene blue, etc.

### (5) Spiro-compounds

3-methylspirodinaphthopyran, 3-ethylspirodinaphthopyran, 3,3-dichlorospirodinaphthopyran, 3-benzylspirodinaphthopyran, 3-methylnaphtho-(3-methoxybenzo)spiropyran, 3-propylspirobenzopyran, etc.

The dye precursor also includes, for example, the following compounds that can absorb a near infrared ray: 3,6-bis(dimethylamino)fluorene-9-spiro-3'-(6'-dimethylaminophthalide), 3-diethylamino-6-dimethylaminofluorene-9-spiro-3'-(6'-dimethylaminophthalide), 3,6-bis(diethylamino)fluorene-9-spiro-3'-(6'-dimethylaminophthalide), 3-dibutylamino-6-dimethylaminofluorene-9-spiro-3'-(6'-dimethylaminophthalide), 3-dibutylamino-6-diethylaminofluorene-9-spiro-3'-(6'-dimethylaminophthalide), 3,6-bis(dimethylamino)fluorene-9-spiro-3'-(6'-diethylaminophthalide), 3-diethylamino-6-dimethylaminofluorene-9-spiro-3'-(6'-diethylaminophthalide), 3-dibutylamino-6-dimethylaminofluorene-9-spiro-3'-(6'-diethylaminophthalide), 3,6-bis(diethylamino)fluorene-9-spiro-3'-(6'-diethylaminophthalide), 3,6-bis(dimethylamino)fluorene-9-spiro-3'-(6'-dibutylaminophthalide), 3-dibutylamino-6-diethylaminofluorene-9-spiro-3'-(6'-diethylaminophthalide), 3-diethylamino-6-dimethylaminofluorene-9-spiro-3'-(6'-dibutylaminophthalide), 3,3-bis[2-(4-dimethylaminophenyl)-2-(4-methoxyphenyl)ethenyl]-4,5,6,7-tetrachlorophthalide, etc.

The urea-urethane compound as developer is used in a proportion of preferably 5 to 1,000 parts by weight, more preferably 20 to 500 parts by weight, per 100 parts by weight of the colorless or light-colored dye precursor. As the proportion of the urea-urethane compound as developer, 5 parts by weight or more is sufficient to allow the dye precursor to develop a color. At such a proportion, the coloring density is high. When the proportion of the urea-urethane compound as developer is 1,000 parts by weight or less, the urea-urethane compound as developer hardly remains as a surplus, and this is economically advantageous and hence preferable.

The incorporation of an isocyanate compound into the color-producing composition of the present invention improves the shelf stability of the composition. The isocyanate compound incorporated into the color-producing composition of the present invention refers to a colorless or light-colored, aromatic or heterocyclic isocyanate compound that is solid at ordinary temperature. For example, one or more of the following isocyanate compounds are used.

The isocyanate compound incorporated includes 2,6-dichlorophenyl isocyanate, p-chlorophenyl isocyanate, 1,3-phenylene diisocyanate, 1,4-phenylene diisocyanate, 1,3-dimethylbenzene-4,6-diisocyanate, 1,9-dimethylbenzene-2,5-diisocyanate, 1-methoxybenzene-2,4-diisocyanate, 1-methoxybenzene-2,5-diisocyanate, 1-ethoxybenzene-2,4-diisocyanate, 2,5-dimethoxybenzene-1,4-diisocyanate, 2,5-diethoxylbenzene-1,4-diisocyanate, 2,5-dibutoxybenzene-1,4-diisocyanate, azobenzene-4,4'-diisocyanate, diphenyl ether-4,4'-diisocyanate, naphthalene-1,4-diisocyanate, naphthalene-1,5-diisocyanate, naphthalene-2,6-diisocyanate, naphthalene-2,7-diisocyanate, 3,3'-dimethyl-biphenyl-4,4'-diisocyanate, 3,3'-dimethoxybiphenyl-4,4'-diisocyanate, diphenylmethane-4,4'-diisocyanate, diphenyldimethylmethane-4,4'-diisocyanate, benzophenone-3,3'-diisocyanate, fluorene-2,7-diisocyanate, anthraquinone-2,6-diisocyanate, 9-ethylcarbazole-3,6-diisocyanate, pyrene-3,8-diisocyanate, naphthalene-1,3,7-triisocyanate, biphenyl-2,4,4'-triisocyanate, 4,4',4"-triisocyanato-2,5-dimethoxytriphenylmethane, 4,4',4"-triisocyanatotriphenylamine, p-dimethylaminophenyl isocyanate, tris(4-phenylisocyanato)thiophosphate, etc. If necessary, these isocyanates may be used in the form of a so-called block isocyanate, i.e., an addition compound with a phenol, lactam, oxime or the like, they may be used in the form of a diisocyanate dimer such as 1-methylbenzene-2,4-diisocyanate dimer, or a diisocyanurate trimer as an isocyanurate, and they may be used in the form of a polyisocyanate obtained as an adduct by the use of any of various polyols and the like. There may also be used water adduct isocyanates of 2,4-toluene diisocyanate, diphenylmethane diisocyanate and the like, such as 1,3-bis(3-isocyanato-4-methylphenyl)urea; polyol adducts such as trimethylolpropane adduct of toluene diisocyanate (Desmodule L, a trade name); phenol adduct isocyanates; amine adduct isocyanates; and the isocyanate compounds and isocyanate adduct compounds described in the specification of JP-A-10-76757 and the specification of JP-A-10-95171.

The isocyanate compound is used in a proportion of preferably 5 to 500 parts by weight, more preferably 20 to 200 parts by weight, per 100 parts by weight of the colorless or light-colored dye precursor. When the proportion of the isocyanate compound is 5 parts by weight or more, a sufficient improving effect on the shelf stability can be obtained and the coloring density is high. When the proportion of the isocyanate compound is 500 parts by weight or less, the isocyanate compound hardly remains as a surplus, and this is economically advantageous and hence preferable.

The incorporation of an imino compound into the color-producing composition of the present invention further improves the shelf stability.

The imino compound that can be incorporated into the color-producing composition of the present invention is a colorless or light-colored compound that has at least one imino group and is solid at ordinary temperatures. Two or more imino compounds may be incorporated in combination, depending on purposes. As the imino compound, those described in JP-A-9-142032 can be mentioned, and the contents of this reference are hereby incorporated herein by reference. Of the imino compounds described in the reference, iminoisoindoline derivatives are preferable, and 1,3-diimino-4,5,6,7-tetrachloroisoindoline, 3-imino-4,5,6,7-tetrachloroisoindolin-1-one and 1,3-diimino-4,5,6,7-tetrabromoisoindoline are more preferable.

The imino compound is used in a proportion of preferably 5 to 500 parts by weight, more preferably 20 to 200 parts by weight, per 100 parts by weight of the colorless or light-colored dye precursor. When the proportion of the imino compound is 5 parts by weight or more, an improving effect on the shelf stability is obtained. When the proportion of the imino compound is 500 parts by weight or less, the imino compound hardly remains as a surplus, and this is economically advantageous and hence preferable.

In addition, the incorporation of an amino compound into the color-producing composition of the present invention improves the preservability of an original recording material surface and print. The amino compound that can be incorporated is a colorless or light-colored substance having at least one primary, secondary or tertiary amino group. As such an amino compound, those described in JP-A-9-142032 can be mentioned. Of the amino compounds described in this reference, aniline derivatives having at least one amino group and represented by the following formula (VIII) are especially preferable: 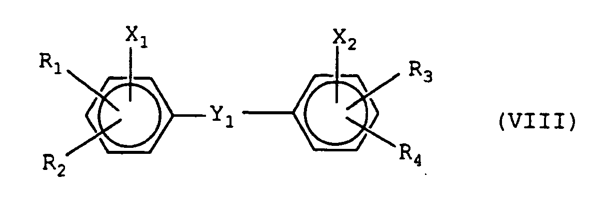 wherein R₁, R₂, R₃ and R₄ are independently a hydrogen atom, a halogen atom, an alkyl group, an alkoxy group or an amino group, X₁ and X₂ are independently an amino group or a group represented by the formula (b): 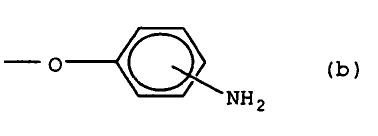 and Y₁ is any of -SO₂-, -O-, -(S)ₙ-, - (CH₂)ₙ-, -CO-, -CONH- and a group represented by any of the formulas (a): 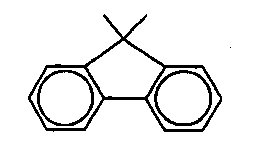 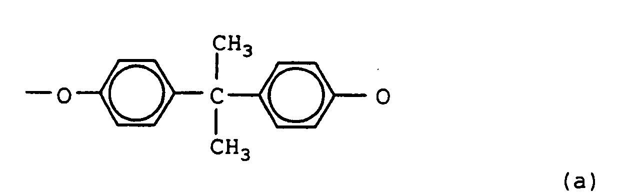 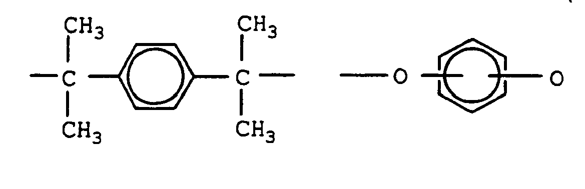 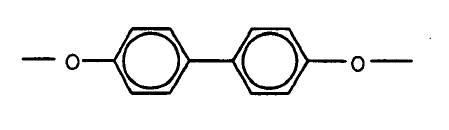 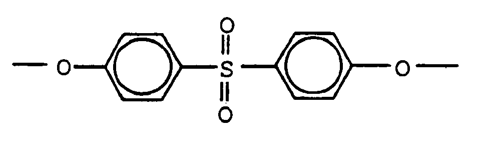 or is absent, and n is 1 or 2.

These amino compounds may be used singly or as a mixture thereof. For improving the print preservability in the plasticizer resistance, the proportion of the amino compound is preferably 1 to 500 parts by weight per 100 parts by weight of the colorless or light-colored dye precursor. When the content of the amino compound is 1 part by weight or more per part of the urea-urethane compound, the print preservability can be improved. When the content is 500 parts by weight or less, performance characteristics of the resulting composition can be sufficiently improved and such a content is advantageous from the viewpoint of cost.

The incorporation of also an acidic developer into the color-producing composition of the present invention improves the sensitivity and enables the color-producing composition to produce a brilliant color.

As the acidic developer that is used when the color-producing composition of the present invention is used in a heat-sensitive recording material, conventional electron-accepting materials are used and, in particular, phenol derivatives; aromatic carboxylic acid derivatives or their metal compounds; salicylic acid derivatives or their metal salts; N,N-diarylthiourea derivatives; sulfonylurea derivatives; etc. are preferable. The phenol derivatives are especially preferable. Specific examples of the phenol derivatives are 2,2-bis(4-hydroxyphenyl)propane, 2,2-bis(hydroxyphenyl)butane, 2,2-bis(hydroxyphenyl)pentane, 2,2-bis(hydroxyphenyl)heptane, 1,1-bis(4-hydroxyphenyl)-cyclohexane, butyl bis(4-hydroxyphenyl)acetate, benzyl bis(4-hydroxyphenyl)acetate, bis(4-hydroxyphenyl) sulfone, bis(3-methyl-4-hydroxyphenyl) sulfone, 4-hydroxyphenyl-4'-methylphenyl sulfone, 3-chloro-4-hydroxyphenyl-4'-methylphenyl sulfone, 3,4-dihydroxyphenyl-4'-methylphenyl sulfone, 4-isopropylphenyl-4'-hydroxyphenyl sulfone, 4-isopropyloxyphenyl-4'-hydroxyphenyl sulfone, bis(2-allyl-4-hydroxyphenyl) sulfone, 4-hydroxyphenyl-4'-benzyloxyphenyl sulfone, 4-isopropylphenyl-4'-hydroxyphenyl sulfone, bis(2-methyl-3-tert-butyl-4-hydroxyphenyl) sulfide, methyl 4-hydroxybenzoate, benzyl 4-hydroxybenzoate, (4'-chlorobenzyl) 4-hydroxybenzoate, ethyl 1,2-bis(4'-hydroxybenzoate), pentyl 1,5-bis(4'-hydroxybenzoate), hexyl 1,6-bis(4'-hydroxybenzoate), dimethyl 3-hydroxyphthalate, stearyl gallate, lauryl gallate, etc. The salicylic acid derivatives include 4-n-octyloxysalicylic acid, 4-n-butyloxysalicylic acid, 4-n-pentyloxysalicylic acid, 3-n-dodecyloxysalicylic acid, 3-n-octanoyloxysalicylic acid, 4-n-octyloxycarbonylaminosalicylic acid, 4-n-octanoyloxycarbonylaminosalicylic acid, etc. The sulfonylurea derivatives include, for example, compounds containing one or more arylsulfonylaminoureido groups, such as 4,4-bis(p-toluenesulfonylaminocarbonylamino)diphenylmethane, 4,4-bis(o-toluenesulfonylaminocarbonylamino)diphenylmethane, 4,4-bis(p-toluenesulfonylaminocarbonylamino)-diphenyl sulfide, 4,4-bis(p-toluenesulfonylaminocarbonylamino)diphenyl ether, N-(p-toluenesulfonyl)-N'-phenylurea, etc.

In order to improve fog, the thermal response and the like, it is also possible to add phenolic compounds such as N-stearyl-N'-(2-hydroxyphenyl)urea, N-stearyl-N'-(3-hydroxyphenyl)urea, N-stearyl-N'-(4-hydroxyphenyl)urea, p-stearoylaminophenol, o-stearoylaminophenol, p-lauroylaminophenol, p-butyrylaminophenol, m-acetylaminophenol, o-acetylaminophenol, p-acetylaminophenol, o-butylaminocarbonylphenol, o-stearylaminocarbonylphenol, p-stearylaminocarbonylphenol, 1,1,3-tris(3-tert-butyl-4-hydroxy-6-methylphenyl)butane, 1,1,3-tris(3-tert-butyl-4-hydroxy-6-ethylphenyl)butane, 1,1,3-tris(3,5-di-tert-butyl-4-hydroxyphenyl)butane, 1,1,3-tris(3-tert-butyl-4-hydroxy-6-methylphenyl)propane, 1,2,3-tris(3-tert-butyl-4-hydroxy-6-methylphenyl)butane, 1,1,3-tris(3-phenyl-4-hydroxyphenyl)butane, 1,1,3-tris(3-cyclohexyl-4-hydroxy-5-methylphenyl)butane, 1,1,3-tris(3-cyclohexyl-4-hydroxy-6-methylphenyl)butane, 1,1,3-tetra(3-phenyl-4-hydroxyphenyl)propane, 1,3,3-tetra(3-cyclohexyl-4-hydroxy-6-methylphenyl)propane, 1,1-bis(3-tert-butyl-4-hydroxy-6-methylphenyl)butane, 1,1-bis(3-cyclohexyl-4-hydroxy-6-methylphenyl)butane, etc.

The above-mentioned acidic developer is used in a proportion of preferably 5 to 500 parts by weight, more preferably 20 to 200 parts by weight, per 100 parts by weight of the colorless or light-colored dye precursor. When the proportion of the acidic developer is 5 parts by weight or more, the color development of the dye precursor is satisfactory and the coloring density is high. When the proportion of the acidic developer is 500 parts by weight or less, the acidic developer hardly remains, and this is economically advantageous and hence preferable.

Also when the color-producing composition of the present invention is used in a pressure-sensitive recording material, the incorporation of an acidic developer into the color-producing composition improves the coloring density and enables the pressure-sensitive recording material to produce a brilliant color.

Also as this acidic developer, an electron-accepting material is used. The acidic developer includes, for example, inorganic compounds such as acid clay, activated clay, attapulgite, bentonite, zeolite, colloidal silica, magnesium silicate, talc, aluminum silicate, etc.; phenol, cresol, butylphenol, octylphenol, phenylphenol, chlorophenol, salicylic acid and the like, or aldehyde condensation novolak resins derived therefrom and their metal salts; and salicylic acid derivatives such as 3-isopropylsalicylic acid, 3-phenylsalicylic acid, 3-cyclohexylsalicylic acid, 3,5-di-t-butylsalicylic acid, 3,5-di(α-methylbenzyl)salicylic acid, 3,5-di-t-octylsalicylic acid, 3-methyl-5-benzylsalicylic acid, 3,5-di(α,α-dimethylbenzyl)salicylic acid, 3-phenyl-5-(α,α-dimethylbenzyl)salicylic, etc. and metal salts thereof.

The color-producing composition of the present invention can be made into a recording material by forming a color-producing layer of the composition on some substrate by a method such as coating. The structure of the recording material is varied depending on the kind of the recording material.

The color-producing composition of the present invention can be used in any of various recording materials such as heat-sensitive recording materials, pressure-sensitive recording materials and the like, and is suitable particularly for the heat-sensitive recording materials.

When the color-producing composition is used in a heat-sensitive recording material, a heat-sensitive recording layer capable of producing a color on heating is formed on a substrate. Specifically, the above-mentioned urea-urethane compound, the above-mentioned colorless or light-colored dye precursor such as a leuco dye, and the heat-meltable material described hereinafter, should be applied on a substrate, each in the form of a dispersion together with other necessary components to form a heat-sensitive recording layer. The dispersion is prepared by finely grinding one or more compounds as each of the components described above, with a sand grinder or the like in an aqueous solution containing a compound having dispersing capability, such as a water-soluble polymer, a surfactant or the like. The particle size of each of the dispersions thus obtained is preferably adjusted to 0.1 to 10 µm, in particular, to about 1 µm. Specific examples of the compound having dispersing capability which can be used in the present invention are water-soluble polymers such as poly(vinyl alcohol)s, carboxylic acid-modified poly(vinyl alcohol)s, sulfonic acid-modified poly(vinyl alcohol)s, methyl cellulose, hydroxypropylmethyl cellulose, hydroxypropyl cellulose, etc.; anionic surfactants such as condensation naphthalene sulfonates, polyoxyethylene alkyl ether sulfuric acid ester salts (e.g. sodium polyoxyethylene lauryl ether sulfates, sodium polyoxyethylene alkyl ether sulfates and sodium polyoxyethylene alkyl phenyl ether sulfates), dialkylsulfosuccinic acid ester sodium, alkylphosphates (e.g. diethanolamine alkylphosphates and potassium alkylphosphates), specialty carboxylic acid-based polymers, etc.; nonionic surfactants such as polyoxyethylene alkyl ethers, polyoxyethylene alky phenyl ethers, polyoxyethylene sorbitan fatty acid esters, fatty acid monoglycerides, polyethylene glycol fatty acid esters, etc.; and cationic surfactants such as dicyanamidopolyamines, tertiary amine salts, quaternary ammonium salts, etc. Of these, the polyvinyl alcohols, carboxylic acid-modified polyvinyl alcohols, sulfonic acid-modified polyvinyl alcohols and methyl cellulose are especially preferable. The above-exemplified surfactants may be used singly or as a mixture thereof. Particularly when the urea-urethane compound is subjected to wet grinding in an aqueous medium, the compound is preferably ground in a neutral pH range of 5 to 10 while maintaining the temperature of the aqueous medium at 60°C or lower. The pH of a coating liquid containing the urea-urethane compound and the colorless or light-colored dye precursor is preferably 5 to 12.

The heat-sensitive recording layer may contain, besides the components described above, pigments such as diatomaceous earth, talc, kaolin, calcined kaolin, calcium carbonate, magnesium carbonate, titanium oxide, zinc oxide, silicon oxide, aluminum hydroxide, urea-formaldehyde resin, etc. In addition, the heat-sensitive recording layer may, if necessary, contain metal salts of higher fatty acids, such as zinc stearate, calcium stearate, etc.; and waxes such as paraffin, oxidized paraffin, polyethylenes, oxidized polyethylenes, stearamide, cator wax, etc., for the purpose of, for example, preventing the wear of a head and sticking. If necessary, the heat-sensitive recording layer may also contain dispersing agents such as sodium dioctylsulfosuccinate, etc.; ultraviolet absorbers of benzophenone type, benzotriazole type and the like; surfactants; fluorescent dyes; etc.

As a binder usable for forming the heat-sensitive recording layer, there can be mentioned, for example, water-soluble binders such as starches, hydroxyethyl cellulose, methyl cellulose, carboxymethyl cellulose, gelatin, casein, poly(vinyl alcbhol)s, modified poly(vinyl alcohol)s, sodium poly(acrylate)s, acrylamide-acrylic ester copolymers, acrylamide-acrylic ester-methacrylic acid terpolymers, alkali salts of styrene-maleic anhydride copolymers, alkali salts of ethylene-maleic anhydride copolymers, etc.; and latex type water-insoluble binders of styrene-butadiene copolymers, acrylonitrile-butadiene copolymers, methyl acrylate-butadiene copolymers, etc.

As the substrate for the heat-sensitive recording layer, paper is mainly used, though any of various woven fabrics, nonwoven fabrics, synthetic resin films, laminated papers, synthetic papers, metal foils, and composite sheets obtained by combining two or more of them may be used besides paper, depending on their purpose. The heat-sensitive recording layer may be composed of either a single layer or two or more layers. The heat-sensitive recording layer may have, for example, a multilayer structure formed by incorporating each color-producing component into one layer. A protective layer composed of a single layer or two or more layers may be formed on the heat-sensitive recording layer, and an intermediate layer composed of a single layer or two or more layers may also be formed between the substrate and the heat-sensitive recording layer. The heat-sensitive recording layer can be obtained by mixing aqueous dispersions prepared by fine grinding of each color-producing component or any other component, with a binder and the like, applying the resulting mixture on the substrate, and drying the mixture. The coating amount of this coating liquid is preferably 1 to 15 g/m² when the coating liquid is in a dried state.

When the color-producing composition of the present invention is used in a heat-sensitive recording material, a heat-meltable material may be incorporated into the color-producing composition in order to improve the sensitivity. The heat-meltable material is preferably one which has a melting point of 60°C to 180°C, in particular, one which has a melting point of 80°C to 140°C. The heat-meltable material includes, for example, benzyl p-benzyloxybenzoate, stearamide, palmitamide, N-methylolstearamide, β-naphthyl benzyl ether, N-stearylurea, N,N'.-distearylurea, phenyl β-naphthoate, phenyl 1-hydroxy-2-naphthoate, β-naphthol (p-methylbenzyl) ether, 1,4-dimethoxynaphthalene, 1-methoxy-4-benzyloxynaphthalene, N-stearoylurea, p-benzylbiphenyl, 1,2-di(m-methylphenoxy)ethane, 1-phenoxy-2-(4-chlorophenoxy)ethane, 1,4-butanediol phenyl ether, dimethyl terephthalate, m-terphenyl, dibenzyl oxalate and (p-chlorobenzyl) oxalate. As the heat-meltable material, there may also be used 4,4'-dimethoxybenzophenone, 4,4'-dichlorobenzophenone, 4,4'-difluorobenzophenone, diphenyl sulfone, 4,4'-dichlorodiphenyl sulfone, 4,4'-difluorodiphenyl sulfone, 4,4'-dichlorodiphenyl disulfide, diphenylamine, 2-methyl-4-methoxydiphenylamine, N,N'-diphenyl-p-phenylenediamine, 1-(N-phenylamino)-naphthalene, benzil, 1,3-diphenyl-1,3-propanedione, etc.

Of these, diphenyl sulfone is preferably used.

The above-exemplified heat-meltable materials may be used singly or as a mixture thereof. For attaining a sufficient thermal response, the heat-meltable material is used in a proportion of preferably 10 to 300 parts by weight, more preferably 20 to 250 parts by weight, per 100 parts by weight of the colorless or light-colored dye precursor.

When the recording material is a pressure-sensitive recording material, it can have, for example, the forms disclosed in U.S. Patent Nos. 2505470, 2712507, 2730456, 2730457 and 3418250, etc. That is, various forms such as the following forms can be employed: pressure-sensitive recording paper obtained by dissolving the dye precursor or a mixture of the dye precursors in a solvent consisting of one of or a mixture of two or more of alkylated naphthalenes, alkylated diphenyls, alkylated diphenylmethanes, alkylated diarylethanes, synthetic oils (e.g. chlorinated paraffin), vegetable oils, animal oils, mineral oils, etc., dispersing the resulting solution in a binder or incorporating the solution into microcapsules, applying the dispersion on a substrate or applying the microcapsules on a substrate together with a binder, and placing the upper paper thus obtained and under paper coated with a dispersion of the urea-urethane compound (and an amino compound and/or a developer, etc.), one upon the other so that their coated surfaces face each other; a pressure-sensitive recording paper obtained by holding, between the above-mentioned upper paper and under paper, an intermediate paper coated with a dispersion of the urea-urethane compound on one side and the dye precursor on the other side; a self-type pressure-sensitive recording paper obtained by applying the above-mentioned dispersion of the urea-urethane compound (and an amino compound and/or a developer) and the above-mentioned dispersion containing the dye precursor, on the same surface of a substrate as a mixture or in a multilayer form; and a self-type pressure-sensitive recording paper obtained by making each of the dye precursor and the urea-urethane compound (and an amino compound and/or a developer) into microcapsules, and applying a mixture of the microcapsules of the two kinds on the same surface of a substrate.

As a process for producing microcapsules, there can be adopted, for example, the coacervation processes disclosed in U.S. Patent Nos. 2800457 and 2800458, the interfacial polymerization processes disclosed in JP-B-38-19574, JP-B-42-446, JP-B-42-771, the in-situ processes disclosed in JP-B-36-9168, JP-B-51-9079, the melt dispersion cooling processes disclosed in Brit. Patent Nos. 952807 and 96-5074, and the spray drying processes disclosed in U.S. Patent No. 311140, Brit. Patent No. 930422.

For forming a pressure-sensitive layer, each component such as the urea-urethane compound may be used in the form of a solution or dispersion in a solvent. In the case of a color-producing system further comprising an amino compound and/or a developer, each component may be used in the form of a solution or dispersion in a solvent, or a combination of the urea-urethane compound, the amino compound and optionally the developer may be used in the form of a solution or dispersion in a solvent.

In the above-mentioned interfacial polymerization processes adopted for forming microcapsules, a film is formed on an interface by using two kinds of monomers, i.e., an oil monomer and a water-soluble monomer. There are known, for example, a process in which a polybasic acid chloride is used as an oil phase and a polyvalent amine as an aqueous phase, and a polyamide film is formed on the interface; a process in which a polybasic acid chloride is used as an oil phase and a polyhydric hydroxy compound as an aqueous phase, and a polyester film is formed on the interface; a process in which a polyvalent isocyanate is used as an oil phase and a polyhydric alcohol or a polyhydric phenol as an aqueous phase, and a polyurethane film is formed on the interface; and a process in which a polyvalent isocyanate is used as an oil phase and a polyvalent amine as an aqueous phase, and a polyurea film is formed on the interface. Thus, when the interfacial polymerization process is adopted for producing microcapsules, an isocyanate compound is used in some cases as a reactive monomer for forming a film.

In this case, said isocyanate compound is consumed in forming a film for microcapsules and is not directly concerned with a developed color image, and it is absolutely necessary to use a water-soluble monomer together with the isocyanate compound. In these points, its employment is distinguished from the employment of the isocyanate compound according to the present invention.

Dispersions of compounds which are not made into microcapsules are prepared by finely grinding one or more compounds as each component in an aqueous solution containing a compound having dispersing capability, such as a water-soluble polymer, a surfactant or the like. The urea-urethane compound may be dispersed together with an amino compound and an acidic developer.

As the substrate used in the pressure-sensitive recording material, paper is mainly used, though any of various woven fabrics, nonwoven fabrics, synthetic resin films, laminated papers, synthetic papers, metal foils, and composite sheets obtained by combining two or more of them may be used besides paper, depending on their purpose.

As a binder, conventional various binders can be used. The binder includes, for example, water-soluble binders such as starches, hydroxyethyl cellulose, methyl cellulose, carboxymethyl cellulose, gelatin, casein, poly(vinyl alcohol)s, modified poly(vinyl alcohol)s, sodium poly(acrylate)s, acrylamide-acrylic ester copolymers, acrylamide-acrylic ester-methacrylic acid terpolymers, alkali salts of styrene-maleic anhydride copolymers, alkali salts of ethylene-maleic anhydride copolymers, etc.; and latex type water-insoluble binders of styrene-butadiene copolymers, acrylonitrile-butadiene copolymers, methyl acrylate-butadiene copolymers, etc.

In the recording material used according to the present invention, the recording layer may contain a hindered phenol compound or an ultraviolet absorber. The hindered phenol compound or ultraviolet absorber includes, for example, 1,1,3-tris(3'-cyclohexyl-4'-hydroxyphenyl)butane, 1,1,3-tris(2-methyl-4-hydroxy-5-tert-butylpheny)butane, 4,4'-thiobis(3-methyl-6-tertbutylphenol), 1,3,5-trimethyl-2,4,6-tris(3,5-di-tert-butyl-4-hydroxybenzyl)benzene, 2,2'-dihydroxy-4,4'-dimethoxybenzophenone, p-octylphenyl salicylate, 2-(2'-hydroxy-5'-methylpheny)benzotriazole, ethyl-2-cyano-3,3'-diphenyl acrylate and tetra(2,2,6,6-tetramethyl-4-piperidyl)-1,2,3,4-butanetetracarbonate.

The present invention is explained in further detail with the following examples.

The analyses of materials and the evaluation of physical properties were carried out by the following methods.

### <IR spectrum>

Measured by diffuse reflectance spectroscopy by the use of FTIR-8100M manufactured by Shimadzu Corp.

### <Mass spectrum>

Measured by using JMS-HX100 manufactured by JEOL LTD., nitrobenzyl alcohol as a matrix, and xenon as a primary gas.

### <Color development sensitivity of thermal paper>

Coloring densiting at an applied voltage of 24 V and a pulse width of 1.5 msec was measured with an optical densitometer by using a printing tester manufactured by Ohkura Denki K.K., and a thermal head KJT-256-8MG manufactured by Kyocera Co., Ltd.

### <Plasticizer resistance>

A heat-sensitive recording material was held between vinyl chloride wrap films or in a vinyl chloride file, and a load of 300 g/cm² was applied thereto from above. After standing at 40°C for 24 hours, the coloring density of the printed portion and the non-printed portion (the original recording material surface) was visually estimated. When there was only a slight decrease in print density, the print preservability was rated as good.

### <Heat resistance>

A heat-sensitive recording material was allowed to stand at 60°C and 25% RH for 24 hours and the degree of fading of print was visually estimated. When the degree of fading is low, the print preservability was rated as good.

In addition, a heat-sensitive recording material was allowed to stand at 80°C and 25% RH for 24 hours and the degree of fading of print was visually estimated. When the degree of fading was low, the print preservability was rated as good. The coloring density of the original recording material surface was also visually estimated. When the color development was slight, the preservability of original recording material surface was rated good.

### <Coloring density of pressure-sensitive paper>

Upper paper and under paper were placed one upon the other so that their coated surfaces might face each other. A pressure was applied thereto from above to obtain a developed color image on the under paper. The color density of the developed color image was measured by means of a densitometer Macbeth RD917.

### <Solvent resistance>

Hand cream (Atrix, a trade name, mfd. by Kao Corp.) was thinly applied on the developed color image portion obtained in the coloring density estimation, and after standing at ambient temperature for 7 days, the color density of print portion was visually estimated. When there was only a slight decrease in a print density, the print preservability was rated as good.

### Example 1

To 88.2 g of 2,4-toluene diisocyanate were added 124 g of methyl ethyl ketone and 15 g of dimethylformamide as solvents, followed by adding dropwise thereto a dilution of 6.3 g of 4,4'-diaminodiphenyl sulfone with a mixture of 25 g of methyl ethyl ketone and 3 g of dimethylformamide, and the reaction was carried out at 25°C for 8 hours. After completion of the reaction, the methyl ethyl ketone was removed by concentration and toluene was added to the residue, and the white solid precipitated was recovered by filtration, washed with toluene and then dried overnight in a vacuum to obtain 10.0 g of a compound as white crystals. Subsequently, 33 g of phenol and 180 g of methyl ethyl ketone were added to 8.4 g of the obtained compound, followed by adding thereto 8.5 mg of triethylamine, and the reaction was carried out at 25°C for 7 hours. After completion of the reaction, toluene was added to the reaction solution and the crystals precipitated were recovered by filtration, washed with toluene and then dried overnight in a vacuum to obtain 10.0 g of a compound as white crystals. Analytical measurements of these white crystals were as follows. Result of IR measurement:
Characteristic peaks appeared at 990 cm⁻¹, 1110 cm⁻¹, 1320 cm⁻¹, 1590 cm⁻¹, 1700 cm⁻¹ and 3350 cm⁻¹. An IR spectrum is shown in Fig. 1. Result of mass spectrum measurement:
[M+H]⁺ was detected at m/z 785.

The structural formula of the major component of this compound is presumed to be the structural formula of the above-mentioned compound (E-24).

Next, 2 g of this compound was ground together with 8 g of a 2.5 wt% aqueous solution of a poly(vinyl alcohol) (Gohseran^{®} L-3266, a trade name, mfd. by The Nippon Synthetic Chemical Industry Co., Ltd.) in a paint shaker for 45 minutes to be dispersed, whereby a dispersion was obtained. The temperature of the dispersion immediately after the dispersing operation was 25°C. The diameter of dispersed particles of the compound was 0.9 µm.

Separately, 70 g of 3-dibutylamino-6-methyl-7-anilinofluoran was ground together with 130 g of a 5.4 wt% poly(vinyl alcohol) aqueous solution in a sand grinder (mfd. by AIMEX CO., LTD.; vessel capacity 400 ml) at a number of revolution of 2,000 rpm for 3 hours to be dispersed, whereby a dispersion was obtained.

Separately, 70 g of diphenyl sulfone was ground together with 130 g of a 5.4 wt% poly(vinyl alcohol) aqueous solution in a sand grinder (mfd. by AIMEX CO., LTD.; vessel capacity 400 ml) at a number of revolution of 2,000 rpm for 3 hours to be dispersed, whereby a dispersion was obtained.

Separately, 10 g of calcium carbonate was mixed with 30 g of water and dispersed by stirring by the use of a stirrer to obtain a dispersion.

A coating liquid was obtained by stirring and mixing the above-mentioned dispersions and other components in the following proportions (dry basis proportions); the dispersion of the above-mentioned compound in terms of dry solids: 20 parts by weight, the diphenyl sulfone dispersion in terms of dry solids: 25 parts by weight, the calcium carbonate dispersion in terms of dry solids: 40 parts by weight, a zinc stearate dispersion (solid content: 16 wt%) in terms of dry solids: 20 parts by weight, and a 15 wt% poly(vinyl alcohol) in terms of dry solids: 15 parts by weight.

The coating liquid was applied on base paper with a basis weight of 50 g/m² by the use of a bar coater of rod number 10 and dried, followed by supercalendering, to obtain a heat-sensitive recording material.

The result of sensitivity evaluation was so good that the optical density was 1.3.

The result of estimating the degree of a thermal color change of the original recording material surface (the heat resistance) was so good that the color change was slight. The thermal fading of the print portion was desirably slight. The result of evaluating the plasticizer resistance by the use of vinyl chloride wrap films was so good that only a slight color change was caused. The results are summarized in Table 1.

### Example 2

To 30 g of 2,4-toluene diisocyanate was added 57 g of methyl ethyl ketone as a solvent, followed by adding dropwise thereto a dilution of 2.15 g of 4,4'-diaminodiphenyl sulfone with 12 g of methyl ethyl ketone, and the reaction was carried out at 50°C for 6 hours. After completion of the reaction, the reaction solution was cooled to room temperature and then toluene was added thereto, and the white solid precipitated was recovered by filtration, washed with toluene and then dried overnight in a vacuum to obtain 4.1 g of a compound as white crystals. Subsequently, 11.8 g of phenol and 78 g of methyl ethyl ketone were added to 3.0 g of the obtained compound, followed by adding thereto 3 mg of triethylamine, and the reaction was carried out at 25°C for 4 hours. After completion of the reaction, toluene was added to the reaction solution and the crystals precipitated were recovered by filtration, washed with toluene and then dried overnight in a vacuum to obtain 3.9 g of a compound as white crystals. The result of IR measurement of these white crystals was the same as in Example 1.

The structural formula of the major component of this compound is presumed to be the structural formula of the above-mentioned compound (E-24).

Next, 2 g of this compound was ground together with 8 g of a 2.5 wt% aqueous solution of a poly(vinyl alcohol) (Gohsenol^{®} KL-05, a trade name, mfd. by The Nippon Synthetic Chemical Industry Co., Ltd.) in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and using p-benzylbiphenyl in place of diphenyl sulfone, and was evaluated. The results obtained are summarized in Table 1.

### Example 3

While stirring 31.5 g of 2,4-toluene diisocyanate at 60°C, a dilution of 21.5 g of 4,4'-diaminodiphenyl sulfone with 120 ml of methyl ethyl ketone was added dropwise thereto over a period of 4 hours, and the reaction was continued at 60°C for another 2 hours. After completion of the reaction, the reaction solution was cooled to room temperature and then toluene was added thereto, and the white solid precipitated was recovered by filtration, washed with toluene and then dried overnight in a vacuum to obtain 47 g of a compound as white crystals. Subsequently, 9.5 g of phenol and 95 ml of methyl ethyl ketone were added to 30 g of the obtained compound, followed by adding thereto 30 mg of triethylamine, and the reaction was carried out at 25°C for 4 hours. After completion of the reaction, toluene was added to the reaction solution and the crystals precipitated were recovered by filtration, washed with toluene and then dried overnight in a vacuum to obtain 38.5 g of a compound as white crystals. The result of IR measurement of these white crystals was the same as in Example 1.

The structural formula of the major component of this compound is presumed to be the structural formula of the above-mentioned compound (E-24).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Example 4

To 10.4 g of 2,4-toluene diisocyanate was added 20 g of methyl ethyl ketone as a solvent, followed by adding thereto a dilution of 3.7 g of 4,4'-diaminodiphenyl sulfone with 30 g of methyl ethyl ketone, and the reaction was carried out at ambient temperature for 20 hours. After completion of the reaction, the methyl ethyl ketone was removed by concentration and then toluene was added to the residue, and the white solid precipitated was recovered by filtration, washed with hexane and then dried overnight in a vacuum to obtain 8.8 g of a compound as white crystals. Subsequently, 15 g of phenol and then a small amount of dibutyltin dilaurate, were added to 4 g of the obtained compound, and the reaction was carried out at 50°C for 4 hours. After completion of the reaction, toluene was added to the reaction solution and the crystals precipitated were recovered by filtration, washed with hexane and then dried overnight in a vacuum to obtain 5.2 g of a compound as white crystals.

The structural formula of the major component of this compound is presumed to be the structural formula of the above-mentioned compound (E-24).

Next, 2 g of this compound was ground together with 8 g of a 2.5 wt% methyl cellulose aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and using benzyloxynaphthalene in place of diphenyl sulfone, and was evaluated. The results obtained are summarized in Table 1.

### Example 5

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, methyl ethyl ketone was added as a solvent to 2 g of the obtained compound, followed by adding thereto 0.9 g of 4,4'-diaminodiphenyl sulfone, and the reaction was carried out at 50°C for 22 hours. The crystals precipitated were recovered by filtration, washed with hexane and then dried overnight in a vacuum to obtain 2.3 g of a compound as white crystals. Analytical measurements of these white crystals were as follows. Result of IR measurement:
Characteristic peaks appeared at 550 cm⁻¹, 1030 cm⁻¹, 1110 cm⁻¹, 1150 cm⁻¹, 1590 cm⁻¹, 1700 cm⁻¹ and 3300 cm⁻¹.

The structural formula of the major component of this compound is presumed to be the structural formula of the above-mentioned compound (E-22).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and using di-p-methylbenzyl oxalate in place of diphenyl sulfone, and was evaluated. The results obtained are summarized in Table 1.

### Example 6

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, methyl ethyl ketone was added as a solvent to 2 g of the obtained compound, followed by adding thereto 0.9 g of 3,3'-diaminodiphenyl sulfone, and the reaction was carried out at 50°C for 22 hours. The crystals precipitated were recovered by filtration, washed with hexane and then dried overnight in a vacuum to obtain 2.5 g of a compound as white crystals. Analytical measurements of these white crystals were as follows.
Result of IR measurement:
Characteristic peaks appeared at 620 cm⁻¹, 880 cm⁻¹, 1030 cm⁻¹, 1300 cm⁻¹, 1590 cm⁻¹, 1700 cm⁻¹ and 3300 cm⁻¹.

The structural formula of the major component of this compound is presumed to be the structural formula of the above-mentioned compound (E-19).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and using benzil in place of diphenyl sulfone, and was evaluated. The results obtained are summarized in Table 1.

### Example 7

To 10.4 g of 2,4-toluene diisocyanate was added 20 g of methyl ethyl ketone as a solvent, followed by adding dropwise thereto a dilution of 3.7 g of 3,3'-diaminodiphenyl sulfone with 30 g of methyl ethyl ketone, and the reaction was carried out at 15°C for 3 hours. The white crystals precipitated were recovered by filtration, washed with hexane and then dried overnight in a vacuum to obtain 6.3 g of a compound as white crystals. Subsequently, 15 g of phenol and then 3 mg of dibutyltin dilaurate were added to 3.0 g of the obtained compound, and the reaction was carried out at 50°C for 3 hours. After completion of the reaction, hexane was added to the reaction solution and the crystals precipitated were recovered by filtration, washed and then dried overnight in a vacuum to obtain 3.3 g of a compound as white crystals. Analytical measurements of these white crystals were as follows.
Result of IR measurement:

Characteristic peaks appeared at 520 cm⁻¹, 880 cm⁻¹, 1030 cm⁻¹, 1430 cm⁻¹, 1590 cm⁻¹, 1710 cm⁻¹ and 3300 cm⁻¹.

The structural formula of the major component of this compound is presumed to be the structural formula of the above-mentioned compound (E-21).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and using 1,2-di(3-methylphenoxy)ethane in place of diphenyl sulfone, and was evaluated. The results obtained are summarized in Table 1.

### Example 8

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, 30 g of toluene was added as a solvent to 2.0 g of the obtained compound, followed by adding thereto 0.41 g of p-phenylenediamine, and the reaction was carried out at 50°C for 10 hours. The crystals precipitated were recovered by filtration, washed with hexane and then dried overnight in a vacuum to obtain 2.3 g of a compound as white crystals. Analytical measurements of these white crystals were as follows.
Result of IR measurement:

Characteristic peaks appeared at 840 cm⁻¹, 1000 cm⁻¹, 1200 cm⁻¹, 1640 cm⁻¹, 1720 cm⁻¹ and 3300 cm⁻¹.

The structural formula of the major component of this compound is presumed to be the structural formula of the above-mentioned compound (E-28).

Next, 2 g of this compound was ground together with 8 g of a 2.5 wt% poly(vinyl alcohol) aqueous solution in a paint shaker for 45 minutes to be dispersed, whereby a dispersion was obtained.

Separately, 70 g of bis(4-hydroxyphenyl) sulfone was ground together with 130 g of a 5.4 wt% poly(vinyl alcohol) aqueous solution in a sand grinder (mfd. by AIMEX CO., LTD.; vessel capacity 400 ml) at a number of revolution of 2,000 rpm for 3 hours to be dispersed, whereby a dispersion was obtained.

Then, a heat-sensitive recording material was produced in the same manner as in Example 1 except for using the dispersion of the compound obtained in Example 24, in place of the dispersion of the compound obtained in Example 1, and adding the bis (4-hydroxyphenyl) sulfone dispersion to the coating liquid in a proportion of 10 parts by weight in terms of dry solids, and this recording material was evaluated. The results obtained are summarized in Table 1.

### Example 9

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, 30 g of dimethylformamide was added as a solvent to 2.7 g of the obtained compound, followed by adding thereto 1.2 g of 4,4'-diaminobenzanilide and then 3 mg of dibutyltin dilaurate, and the reaction was carried out at 25°C for 24 hours. Methanol was poured into the reaction solution, and the crystals precipitated were recovered by filtration, washed and then dried overnight in a vacuum to obtain 2.3 g of a compound as white crystals.

The structural formula of the major component of this compound is presumed to be the structural formula of the above-mentioned compound (E-26).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Example 10

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, 30 g of toluene was added as a solvent to 2.0 g of the obtained compound, followed by adding thereto 0.75 g of 4,4'-diaminodiphenyl ether, and the reaction was carried out at 50°C for 16 hours. The crystals precipitated were recovered by filtration, washed with hexane and then dried overnight in a vacuum to obtain 2.4 g of a compound as white crystals.

The structural formula of the major component of this compound is presumed to be the structural formula of the above-mentioned compound (E-25).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Example 11

To 30 g of 2,4-toluene diisocyanate was added 30 g of toluene as a solvent, followed by adding thereto 3.24 g of phenol, and the reaction was carried out at 100°C for 1 hour and 30 minutes. After completion of the reaction, the toluene was removed by concentration and hexane was added to the residue, and the white solid precipitated was recovered by filtration, washed with hexane and then dried overnight in a vacuum to obtain 6.9 g of a compound as white crystals. Subsequently, 30 g of toluene was added as a solvent to 2.0 g of the obtained compound, followed by adding thereto 0.74 g of 4,4'-diaminodiphenylmethane and 10 g of methyl ethyl ketone, and the reaction was carried out at 50°C for 10 hours. The crystals precipitated were recovered by filtration, washed with hexane and then dried overnight in a vacuum to obtain 2.1 g of a compound as white crystals.

The structural formula of the major component of this compound is presumed to be the structural formula of the above-mentioned compound (E-27).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Example 12

While stirring 150 g of 2,4-toluene diisocyanate at 50°C, a solution of 17.2 g of 4,4'-diaminodiphenylmethane in 120 ml of methyl ethyl ketone was added dropwise thereto over a period of 4 hours, and the reaction was continued at 50°C for another 2 hours. After completion of the reaction, the reaction solution was cooled to room temperature and then toluene was added thereto, and the white solid precipitated was recovered by filtration, washed with toluene and then dried overnight in a vacuum to obtain 43 g of a compound as white crystals. Subsequently, 51.5 g of phenol and 100 ml of methyl ethyl ketone were added to 30 g of the obtained compound, followed by adding thereto 30 mg of triethylamine, and the reaction was carried out at 50°C for 20 hours. After completion of the reaction, toluene was added to the reaction solution and the crystals precipitated were recovered by filtration, washed with toluene and then dried overnight in a vacuum to obtain 38.5 g of a compound as white crystals.

The structural formula of the major component of this compound is presumed to be the structural formula of the above-mentioned compound (E-27).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Example 13

While stirring 31.5 g of 2,4-toluene diisocyanate at 60°C, a solution of 21.5 g of 4,4'-diaminodiphenyl sulfone in 120 ml of methyl ethyl ketone was added dropwise thereto over a period of 6 hours. Uninterruptedly, the reaction solution was cooled to 25°C, followed by adding thereto 17.1 g of phenol and then 30 mg of triethylamine, and the reaction was carried out at 25°C for 4 hours. After completion of the reaction, the methyl ethyl ketone was distilled off and the solid thus obtained was ground and then dried overnight in a vacuum to obtain 70 g of a compound as light-yellow powder.

The structural formula of the major component of this compound is presumed to be the structural formula of the above-mentioned compound (E-24).

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Comparative Example 1

A heat-sensitive recording material was produced in the same manner as in Example 1 except for using 2,2-bis(4-hydroxyphenyl)propane in place of the urea-urethane compound synthesized in Example 1, and was evaluated. The results obtained are summarized in Table 1.

### Comparative Example 2

To 10 g of 2,4-toluene diisocyanate was added 50 g of toluene as a solvent, followed by adding thereto 30 g of aniline, and the reaction was carried out at 25°C for 3 hours. After completion of the reaction, the white solid precipitated was recovered by filtration, washed with hexane and then dried overnight in a vacuum to obtain 17 g of a compound as white crystals.

The presumed structural formula of the major component of this compound is the structural formula of the compound (C-1) shown below.

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Comparative Example 3

To 10 g of 2,4-toluene diisocyanate was added 30 g of toluene as a solvent, followed by adding thereto 30 g of phenol, and the reaction was carried out at 100°C for 3 hours. After completion of the reaction, the toluene was removed by concentration and hexane was added to the residue, and the white solid precipitated was recovered by filtration, washed with hexane and then dried overnight in a vacuum to obtain 15 g of a compound as white crystals.

The presumed structural formula of the major component of this compound is the structural formula of the compound (C-2) shown below.

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

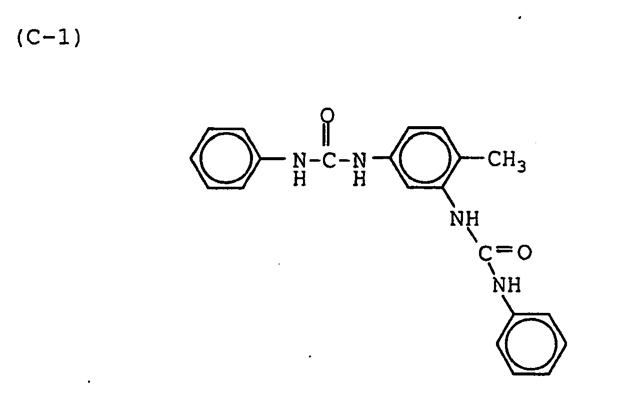

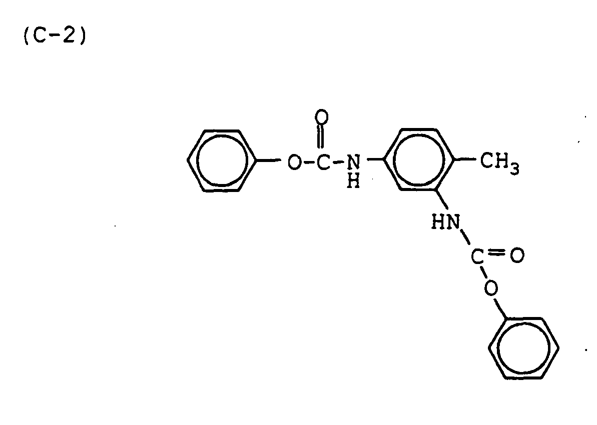

### Comparative Example 4

A heat-sensitive recording material was produced in the same manner as in Example 1 except for using 1,3-diphenylurea in place of the urea-urethane compound synthesized in Example 1, and was evaluated. The results obtained are summarized in Table 1.

**[Table 1]**

| | Presumed structural formula | Sensitivity of thermal paper | Plasticizer resistance (print pre-servability) | Heat resistance | | | Total evaluation |
|---|---|---|---|---|---|---|---|
| | | | | Print pre-servability at 60°C | Print pre-servability at 80°C | Preservability of original recording material surface at 80°C | |
| Example 1 | E-24 | 1.3 | ⊚ | ⊚ | ⊚ | ○ | ⊚ |
| Example 2 | E-24 | 1.3 | ⊚ | ⊚ | ⊚ | ○ | ⊚ |
| Example 3 | E-24 | 1.3 | ⊚ | ⊚ | ⊚ | ○ | ⊚ |
| Example 4 | E-24 | 1.3 | ⊚ | ⊚ | ⊚ | ○ | ⊚ |
| Example 5 | E-22 | 1.3 | ○ | ⊚ | ⊚ | ○ | ○∼⊚ |
| Example 6 | E-19 | 1.3 | ⊚ | ⊚ | ⊚ | ○ | ⊚ |
| Example 7 | E-21 | 1.3 | ○ | ⊚ | ⊚ | ○ | ○∼⊚ |
| Example 8 | E-28 | 1.0 | ○ | ○ | ○ | ○ | ○ |
| Example 9 | E-26 | 1.0 | ○ | ⊚ | ⊚ | ○ | ○∼⊚ |
| Example 10 | E-25 | 1.1 | ○ | ⊚ | ○ | ○ | ○ |
| Example 11 | E-27 | 1.1 | ○ | ⊚ | ○ | ⊚ | ○∼⊚ |
| Example 12 | E-27 | 1.2 | ○ | ○ | ○ | ⊚ | ○ |
| Example 13 | E-24 | 1.3 | ⊚ | ⊚ | ⊚ | ○ | ⊚ |
| Comparative Example 1 | | 1.3 | × | ○ | △ | × | × |
| Comparative Example 2 | C-1 | 0.5 | × | × | × | ○ | × |
| Comparative Example 3 | C-2 | 0.4 | × | × | × | ○ | × |
| Comparative Example 4 | | 0.6 | × | × | × | △ | × |

1. Sensitivity becomes higher with an increase of optical density (OD value).
2. Plasticizer resistance (print preservability)
   ⊚ ∼ Substantially no fading.
   ○ ∼ A slight color tone change without blur and the like.
   △ ∼ Marked fading.
   × ∼ Complete loss of the color of print.
3. Heat resistance (print preservability at 60°C and 80°C)
   ⊚ ∼ Substantially no fading.
   ○ ∼ A slight color tone change without blur and the like.
   △ ∼ Marked fading.
   × ∼ Complete loss of the color of print.
4. Heat resistance (the preservability of an original recording material surface at 80°C)
   ⊚ ∼ Substantially no fog was caused.
   ○ ∼ Reading of a print portion was possible though there was a slight color tone change.
   △ ∼ Reading of a print portion was difficult owing to fog.
   × ∼ Reading of a print portion was impossible owing to serious fog..

### Example 14

### (1) Production of upper paper

A solution prepared by dissolving 2.5 parts by weight of 3-diethylamino-7-chlorofluoran in 80 parts by weight of NISSEKI HISOL^{®} N-296 (an oil, a trade name, mfd. by Nippon Sekiyu Kagaku K.K.) was emulsified in 100 parts by weight of a 5% aqueous solution of pH 4.0 prepared by dissolving a styrene-maleic anhydride copolymer together with a small amount of sodium hydroxide. On the other hand, when a mixture of 10 parts by weight of melamine, 25 parts by weight of a 37% aqueous formaldehyde solution and 65 parts by weight of water was adjusted to pH 9.0 with sodium hydroxide and heated at 60°C, the mixture became transparent after 15 minutes and a melamine-formaldehyde precondensate was obtained. The precondensate was added to the emulsion obtained above, and the resulting mixture was continuously stirred for 4 hours while maintaining the mixture at 60°C, and then was cooled to room temperature. The solid content of the resulting microcapsule dispersion was 45%.

The thus obtained microcapsule dispersion was applied on paper and dried to obtain upper paper.

### (2) Production of under paper

At ordinary temperature, 15 g of a compound (the presumed structural formula of the major component of this compound is the structural formula of the above-mentioned compound (E-22)) synthesized in the same manner as in Example 4 was ground together with 45 g of a 2 wt% poly(vinyl alcohol) aqueous solution in a paint shaker for 45 minutes to be dispersed, whereby a dispersion was obtained.

Separately, 60 g of calcium carbonate was mixed with 90 g of water and dispersed with a stirrer to obtain a dispersion.

A coating liquid was prepared by mixing and stirring 40 parts by weight of the dispersion of the aforesaid synthesized compound, 125 parts by weight of the calcium carbonate dispersion and 120 parts by weight of a 10 wt% poly(vinyl alcohol) aqueous solution.

The coating liquid was applied on base paper with a basis weight of 40 g/m² by the use of a bar coater of rod number 10 to obtain under paper.

The result of evaluating the solvent resistance by the use of hand cream was so good that reading of a print portion was possible. The results are summarized in Table 2.

### Comparative Example 5

A pressure-sensitive recording material was produced in the same manner as in Example 14 except for using activated clay as developer in place of the urea-urethane compound used in Example 14, and was evaluated. The results are summarized in Table 2.

**[Table 2]**

| | Coloring density of pressure-sensitive paper | Solvent resistance (hand cream) |
|---|---|---|
| Example 14 | 1.3 | ⊚ |
| Comparative Example 5 | 0.9 | × |

1. Coloring density becomes higher with an increase of optical density (OD value).
2. Solvent resistance (hand cream)
   ⊚ ∼ Substantially no fading.
   ○ ∼ A slight color tone change without blur and the like.
   △ ∼ Marked fading.
   × ∼ Complete loss of the color of print.

### INDUSTRIAL APPLICABILITY

Employment of a specific urea-urethane compound makes it possible to provide at a low price a color-producing composition and a recording material which are excellent in image preservability and color development sensitivity.

## Claims

1. A urea-urethane compound represented by the following formula (III): 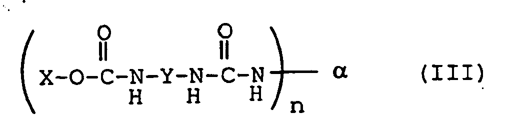 wherein X is a phenyl group, Y is a tolylene group or a methylenediphenyl group represented by -Ph-CH₂-Ph-, α is a residue having a valence of 2 represented by -Ph-or -Ph-ε-Ph-, and n is 2, wherein -Ph- is a phenylene group and -ε- is a group selected from -SO₂-, -O-, -CH₂- and -CONH-.

2. A color-producing composition comprising a developer comprising a urea-urethane compound, and a colorless or light-colored dye precursor, wherein said developer is a urea-urethane compound according to claim 1.

3. A color-producing composition according to claim 2, wherein the developer further comprises an acidic developer.

4. Use of the color-producing composition according to claim 2 or 3 for the production of a heat-sensitive recording material.

## Patentansprüche

1. Harnstoff-Urethan-Verbindung der folgenden Verbindung (III): 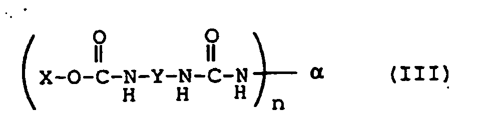 worin X eine Phenylgruppe ist, Y eine Tolylengruppe oder eine durch -Ph-CH₂-Ph- dargestellte Methylendiphenylgruppe ist, α ein Rest mit einer Wertigkeit von 2, dargestellt durch -Ph- oder -Ph-ε-Ph- ist, und n 2 ist, worin -Ph- eine Phenylengruppe ist und -ε- eine aus -SO₂- -O-, -CH₂- und -CONH- ausgewählte Gruppe ist.

2. Farbbildende Zusammensetzung, welche einen Entwickler, der eine Harnstoff-Urethan-Verbindung umfasst, enthält, sowie einen farblosen oder schwach gefärbten Farbstoffvorläufer, worin der Entwickler eine Harnstoff-Urethan-Verbindung nach Anspruch 1 ist.

3. Farbbildende Zusammensetzung nach Anspruch 2, worin der Entwickler weiterhin einen sauren Entwickler umfasst.

4. Verwendung der farbbildenden Zusammensetzung nach Anspruch 2 oder 3 zur Herstellung eines wärmeempfindlichen Aufzeichnungsmaterials.

## Revendications

1. Composé d'urée-uréthane représenté par la formule (III) suivante : 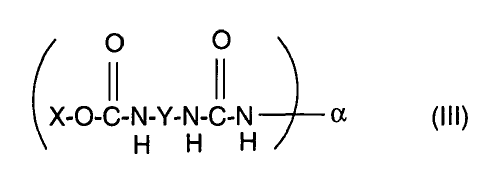 dans laquelle X est un groupe phényle, Y est un groupe tolylène ou un groupe méthylènediphényle représenté par -Ph-CH₂-Ph-, α est un résidu ayant une valence de 2 représenté par -Ph- ou - Ph-ε-Ph- et, n est égal à 2, dans lequel -Ph- est un groupe phénylène et -ε- est un groupe choisi parmi -SO₂-, -O-, -CH₂- et -CONH-.

2. Composition produisant de la couleur comprenant un révélateur comprenant un composé d'urée-uréthane, et un précurseur de colorant sans couleur ou légèrement coloré, dans laquelle ledit révélateur est un composé d'urée-uréthane selon la revendication 1.

3. Composition produisant de la couleur selon la revendication 2, dans laquelle le révélateur comprend de plus un révélateur acide.

4. Utilisation de la composition produisant de la couleur selon la revendication 2 ou 3 pour la production d'un matériau d'enregistrement thermo-sensible.
